(19) **Europäisches Patentamt**
**European Patent Office**
**Office européen des brevets**

(11) **EP 1 988 844 B1**

(12) **EUROPEAN PATENT SPECIFICATION**

(45) Date of publication and mention
of the grant of the patent:
**29.08.2012 Bulletin 2012/35**

(51) Int Cl.:
**A61B 19/00** (2006.01)

(21) Application number: **07706152.1**

(22) Date of filing: **15.02.2007**

(86) International application number:
**PCT/IL2007/000214**

(87) International publication number:
**WO 2007/094001 (23.08.2007 Gazette 2007/34)**

(54) **IMPLANTABLE MEDICAL MARKER AND METHODS OF PREPARATION THEREOF**

IMPLANTIERBARER MEDIZINISCHER MARKER UND HERSTELLUNGSVERFAHREN DAFÜR

MARQUEUR MÉDICAL IMPLANTABLE ET MÉTHODES POUR SA PRÉPARATION

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **16.02.2006 US 773931 P
16.02.2006 US 773930 P
08.06.2006 US 804178 P
10.08.2006 PCT/IB2006/052770
10.08.2006 PCT/IB2006/052771**

(43) Date of publication of application:
**12.11.2008 Bulletin 2008/46**

(73) Proprietor: **Navotek Medical Ltd.
20692 Yokneam (IL)**

(72) Inventors:
• **NEUSTADTER, David, Maier
42756 Natania (IL)**

• **KORNBLAU, Giora
30500 Binyamina (IL)**

(74) Representative: **Fichter, Robert Arno
Dennemeyer & Associates S.A.
55, rue des Bruyères
1274 Howald (LU)**

(56) References cited:
**EP-A- 0 466 681       EP-A- 0 993 843
EP-A- 1 060 764       WO-A-00/24332
WO-A-00/71204       WO-A-01/30447
WO-A-01/87409       WO-A-99/21615
WO-A-03/011161       WO-A-2004/026111
WO-A-2006/004542       WO-A-2006/016368
US-A- 5 342 283       US-A- 5 460 592
US-A1- 2001 005 930       US-A1- 2002 058 853
US-A1- 2002 087 078       US-A1- 2003 088 140
US-A1- 2004 068 157       US-B1- 6 264 599**

## Description

## FIELD OF THE INVENTION

The invention relates generally to implantable markers which can be located using a tracking device and/or seen in a medical image.

## BACKGROUND OF THE INVENTION

Medical markers typically are either visible in medical imaging and/or emit a signal detectable by a dedicated detection device. In some cases, medical imaging provides a relative location of the marker with respect to an anatomic location, but does not provide an absolute location of the marker in terms of position co-ordinates. In other cases, a dedicated detection device provides an absolute location of the marker in terms of position co-ordinates but does not provide a relative position with respect to an anatomic location. Registration of relative and absolute location can be problematic.

BRACHYTHERAPY SEED DESIGNS

In brachytherapy, ionizing radiation is applied to a target for therapeutic purposes by implantation of a brachytherapy "seed" which produces cytotoxic ionizing radiation. The seed is implanted within the body in proximity to the target.

US 6,436,026 to Sioshani (RadioMed Corp.) and US 2004/0116767 by Lebovic disclose spiral configuration brachytherapy seeds. The Lebovic application discloses delivery of the seed via a needle.

WO 02/078785 by Radiovascular Inc.; WO 2004/026111 by Microsperix LLC.; US 6,749,555 to Winkler (Proxima Therapeutics inc.); US 2003/0158515 by Gonzalez (Spiration Inc.) each disclose brachytherapy seed designs which anchor themselves within the body.

SYSTEMS INCLUDING DEDICATED DETECTION DEVICES

US 4,215,694 to Isakov teaches a device for tracking the position of an irradiated object and an electromechanical drive unit for aiming a beam source. The device for tracking the position relies upon sensors in the form of pulse transformers.

WO015476 by ZMED teaches a system for aiming a radiation beam by aligning a frame (bed) holding a patient.

IMPLANTABLE MARKERS FOR POSITION DETERMINATION

US 2005/0261570 by Mate teaches implantation of excitable markers in/near a target. An external excitation source is then aimed at the marker to excite it. The excitation energy is used for position determination. Therapeutic radiation is aimed at a position determined by the marker excitation energy.

US 2005/0027196 by Fitzgerald teaches a system for processing patient radiation treatment data, Fitzgerald teaches use of imaging equipment to determine positions of brachytherapy radiation sources implanted in a patient.

WO 00/57923 teaches a radioactive seed which discloses the orientation and location of the seed when exposed to X-ray. Orientation is indicated by use of different radio-opaque materials.

US 2005/0197564 by Dempsey teaches use of MRI to identify where tracer is taken up, as ionizing radiation is applied.

A series of US patents assigned to Calypso Medical Technologies (e.g. US 6,977,504; US 6,889,833; US 6,838,990; US 6,822,570 and US 6,812,842) describe use of AC electromagnetic localization transponders in conjunction with a position determination system.

LOCATION DETERMINATION BY MONITORING INTRABODY RADIATION

Co-pending PCT application WO 2006/016368 by the inventors of the present invention teaches the use of multiple directional sensors for real time measurement of the 3 dimensional position of a gamma emitting source.

US 6,603,124 to Maublant teaches the use of a directional sensor for detecting a direction towards a gamma emitting source.

Document WO 2006/004542 discloses a marker which folds upon itself according to the preamble of claim 1.

Document WO 00/24332 discloses a self anchoring marker in the form of a coil. The marker is biased to expand after it is expelled from a delivery tube, but it does not fold upon itself.

MARKERS VISIBLE IN MEDICAL IMAGING

Soft tissue markers which can be visualized using medical imaging are described in, for example, US patents 6,575,991; 6,228,055; 6,425,903; 6,056,700; 6,234,177; 6,181,960; 6,662,041 and 6,862470.

The list does not purport to be exhaustive. Markers which can be visualized using medical imaging can be constructed of metal and/or polymers and/or gels. Various markers described in these patents are visible in one or more of Ultrasound, X-ray, CT, and MRI images. Some of these markers can be implanted through very narrow needles (25 gauge or narrower), although most of them are designed for implantation through large bore biopsy needles (13 or 14 gauge). Some of these markers have specific features intended to reduce migration, such as bioadhesive material and/or mechanical hook and/or mechanical flexibility.

IMPLANTABLE BRACHYTHERAPY SOURCES

US patents 6,132,359; 5,713828; 5,997463; 6,419,621; 6,986,880; 6,575,888 and 7,083,566 describe implants made of metal, polymers, and micro sphere containing gels with radioactive material incorporated into the implant. The list does not purport to be exhaustive. Many of these patents describe implants with features intended to reduce migration and to increase visibility in one or more medical imaging modalities. The described markers include a cyto-toxic amount of radiation intended for therapy (i.e. brachytherapy). As a group, implants described in these patents are adapted to provide high levels of localized radiation in a form which is absorbed by adjacent tissue with only a small amount of radiation reaching distant organs or escaping the body.

These implanted sources typically rely upon beta emissions or low energy (below 100keV) gamma emissions. The energy from low energy gamma emissions is primarily absorbed within a few centimeters as they pass through soft tissue. The energy from beta emissions is primarily absorbed within a few millimeters as they pass through soft tissue. The absorption transfers the emitted energy to the tissue and produces a local cytotoxic effect without exposing the rest of the patient's body and/or other people to ionizing radiation. As a result, these implanted sources are generally not well suited for detection, location determination or tracking by a radiation sensor or detector located outside the body.

VASO-OCCLUSIVE DEVICES

US patent 6,616,591 describes a radioactive polymer which can be used together with vaso-occlusive devices to render them radioactive. This patent describes use of the polymers in conjunction with devices designed for insertion into vessels within the body via a catheter.

**SUMMARY OF THE INVENTION**

The invention relates to an implantable medical marker which includes a radioactive source. Visibility is improved by expansion of the marker as, or after, it exits an implantation tool. This expansion may contributes to a reduction in a tendency of the marker to migrate.

Optionally, the radioactive source has a clinically insignificant effect on surrounding tissue but produces a detectable radioactive signal outside the body. In an exemplary embodiment of the invention, the detectable radioactive signal comprises at least 400,000 emitted photons per second that escape the patient's body. The detectable radioactive signal may be used for location determination and/or tracking.

The marker is adapted for injection via a narrow needle. The narrow needle is a 27, 25, 23 or 20 gauge or intermediate gauge. In an exemplary embodiment of the invention, the marker is configured and/or formed of suitable materials so as to be visible in a medical image such as, for example an X-ray image, a CT image, an MRI image or an ultrasound image.

The marker is compressed to conform to an internal volume of the injection needle and expands to define a larger volume when injected into soft tissue. In an exemplary embodiment of the invention, the defined larger volume is filled to a sufficient degree by the marker to make the defined larger volume visible in the medical image. Optionally, filling to a sufficient degree refers to 5, 10, 15, 25 or 50% or lesser or intermediate or larger percentages of the defined volume.

The implantable radioactive medical marker may be adapted for implantation via a 20 gauge tool (e.g. needle) and further adapted to define a three dimensional volume having a smallest dimension of 2-5mm after implantation in tissue. The combination of implantation via a 20 gauge needle and definition of a volume having a smallest dimension of 2-5mm after implantation in tissue requires that the marker expand and/or change shape and/or deform upon implantation. The three dimensional volume(s) defined by the marker upon implantation in tissue comprise a 2-5 mm spheroid or ellipsoid.

In an exemplary embodiment of the invention, the marker comprises a chain which folds on itself upon implantation to define a 2-5 mm spheroid. Optionally, the spheroid comprises a plurality of shapes, optionally irregular shapes. The shapes may increase the visibility of the implanted marker in a selected imaging mode. The shapes may contribute to an increase in surface area which contributes to a reduction in a tendency to migrate within a tissue. Alternatively, or additionally, an increase in surface area increases visibility of the marker in ultrasound imaging. Optionally, the marker is formed of a material and/or constructed so that it does not cause artifacts in an MRI image.

In an exemplary embodiment of the invention, the marker has a relatively uniform spatial distribution of radioactivity. Alternatively or additionally, the implanted marker has a radio-opaque aspect of sufficient size and density to be visible in X-ray and/or CT imaging in all orientations. "Radio-opaque" as used in this specification and the accompanying claims indicates absorption of at least 1% of an incident X-ray beam. Optionally, exemplary markers according to the invention achieve 1,2,5,10 or 15% absorption or intermediate or greater levels of absorption.

According to the invention a gamma emitting radioisotope in a coil shaped implantable medical device is used. The coiled configuration may reduce migration. Optionally, the coiled shape has substantially no elastic memory.

In an exemplary embodiment of the invention, the radioactive source is supplied as an approximately spherical adhesive drop with a diameter of between 2.0 and 5.0 mm.

In an exemplary embodiment of the invention, the marker includes a fixation element integrally formed with or attached to the radiation source. Optionally, the fixation element is adapted to prevent migration and/or unwanted dispersal of the source within the body. Optionally, the fixation element employs a physical configuration and/or an adhesive material and/or a coating to make the source self anchoring.

Optionally, the marker includes a radio-opaque portion. In an exemplary embodiment of the invention, the radio-opaque portion allows visualization of the marker using X-ray based imaging methods. Optionally, visualization is useful during placement of the marker near a target.

An aspect of some embodiments of the present invention relates to a kit including an implantable marker as described above together with an implantation needle adapted to contain the marker and an ejection tool adapted to expel the marker from the injection needle. In an exemplary embodiment of the invention, the marker is inserted into the implantation needle at a manufacturing facility. Optionally, the ejection tool is inserted into the implantation needle at a manufacturing facility.

For purposes of this specification and the accompanying claims, the terms "migrate" and "migration" mean shifting of a center of mass of the radioactive marker relative to a defined location in the surrounding tissue. Optionally, migration is measured only after a first image has been acquired or a first measurement has been made. The phrase "additional migration" indicates migration which occurs after a selected period of time after placement of the marker.

As an illustrative example, if a marker containing a radio-opaque element and a radiation source is implanted a week before it is intended to be used as a marker during a medical treatment, the marker may be subject to migration prior to the treatment. Optionally, this migration ceases or becomes negligible as a result of setting and/or hardening and/or adhesion and/or tissue in-growth. Prior to beginning the treatment, an image is acquired (e.g. X-ray or CT) which shows the relative positions of the centers of mass of the marker and the target. These relative positions may then be used to calculate the target position based on the marker position. In an exemplary embodiment of the invention, little or no additional migration occurs after this stage and throughout the treatment.

Implantable radioactive markers as described above may be employed in one or more of tracking of items, mapping, aiming of external devices, monitoring of tumor and/or therapy progression, and positioning a target with respect to an external reference frame.

Expansion of the marker as it exits an injection tool contributes to an ability to use a narrower gauge tool and/or to improved marker visibility after implantation and/or to reduced radiation dose to the tissue surrounding the marker.

The term "expansion" as used in this specification and the accompanying claims includes, but is not limited to, deformation and/or reshaping. In an exemplary embodiment of the invention, the marker expands in an irregular fashion to a disorganized form which defines a volume.

The term "disorganized" as used in this specification and the accompanying claims indicates one or more of disordered, chaotic and irregular. Optionally, two identical markers injected into similar tissue are characterized by non-identical disorganized forms after expansion.

Optionally, a marker includes one or more disorganized portions prior to insertion. In an exemplary embodiment of the invention, these disorganized portions are compressed during insertion into a needle.

According to the invention, there is provided an implantable medical marker, the marker comprising:

> (a) a marker body adapted for insertion via a needle and adapted to define a volume with a smallest dimension larger than an inner diameter of the needle; and
> (b) a radiation source- characterized by gamma emissions sufficient to exit the human body.

The smallest dimension is 2-5mm

Optionally, the gamma emissions produce between $1 \times 10^5$ and $3 \times 10^8$ photons/second.

Optionally, the gamma emissions produce not more than $5 \times 10^7$ photons/second.

Optionally, the gamma radiation is characterized by an average energy of at least 50 kev.

Optionally, the gamma radiation is characterized by an average energy of at least 150 kev.

Optionally, the gamma radiation is characterized by an average energy not exceeding 400 kev.

Optionally, the gamma radiation is characterized by an average energy not exceeding 1000 kev.

Optionally, the marker is characterized by at least 1% absorption of an incident X-ray beam on the defined volume.

Optionally, the marker produces a radiation dose not exceeding 100Gy at a distance of 2mm from the marker in 6 months.

Optionally, the marker produces a radiation dose not exceeding 40Gy at a distance of 2mm from the marker in 6 months.

Optionally, marker body includes one or more disorganized sections.

Optionally, the defmed volume is only partially occupied by the marker body.

Optionally, the marker body is disorganized.

Optionally, the marker body is jumbled.

Optionally, the marker body is random.

Optionally, the marker body is chaotic.

Optionally, a center of opacity and a center of radioactivity are both within the defined volume.

Optionally, a center of opacity and a center of radioactivity are in a defined spatial relationship with respect to one another.

Optionally, a center of opacity and a center of radioactivity are spaced apart less than 20% of the largest dimension of the defmed volume.

Optionally, the marker is characterized by a spherically uniform distribution of radiation emission within 30%.

. Optionally, the marker is characterized in that at least a portion of the marker is adapted to absorb between 2 and 25 percent of 70kev X-ray radiation incident on the marker.

Optionally, the marker is adapted for insertion via a needle a 21 gauge needle.

Optionally, the marker body is adapted for insertion via a needle a 23, 25 or 27 gauge needle.

Optionally, the marker body is constructed of a radio-opaque radioactive metal.

Optionally, the marker body comprises a radio-opaque radioactive wire with a spring-like memory.

The marker body comprises a coil.

Optionally, the coil includes a Platinum/Iridium alloy.

The coil is adapted for folding.

According to the invention, there is also provided kit comprising a marker as described and an insertion tool.

Optionally, the kit comprises an ejection tool.

Optionally, the insertion tool comprises a needle.

Optionally, the implanted marker is provided loaded into the insertion tool.

According to the invention, there is also provided a method of preparing an implantable medical marker as described above, the method comprising:

> (a) associating an amount of gamma radiation with a metal wire;
> (b) forming the wire into a desired shape; and
> (c) inserting the wire in a needle.

BRIEF DESCRIPTION OF THE DRAWINGS

Exemplary non-limiting embodiments of the invention are described in the following description, read with reference to the figures attached hereto. In the figures, identical and similar structures, elements or parts thereof that appear in more than one figure are generally labeled with the same or similar references in the figures in which they appear. Dimensions of components and features shown in the figures are chosen primarily for convenience and clarity of presentation and are not necessarily to scale. The attached figures are:

Fig. 1 is a simplified flow diagram of a method of using an implantable medical marker;

Fig. 2a is a side view of an implantable medical marker;

Fig. 2b is a lateral cross section of the implantable medical marker of Fig. 2A loaded into an implantation tool;

Fig. 3a is a side view of an implantable medical marker;

Fig. 3b is a lateral cross section of the implantable medical marker of Fig. 2C loaded into an implantation tool;

Figs. 4 and 5 are side views of different implantable medical markers;

Fig. 6 is a schematic representation of an exemplary helical marker body illustrating exemplary dimensions.

## DETAILED DESCRIPTION

Overview

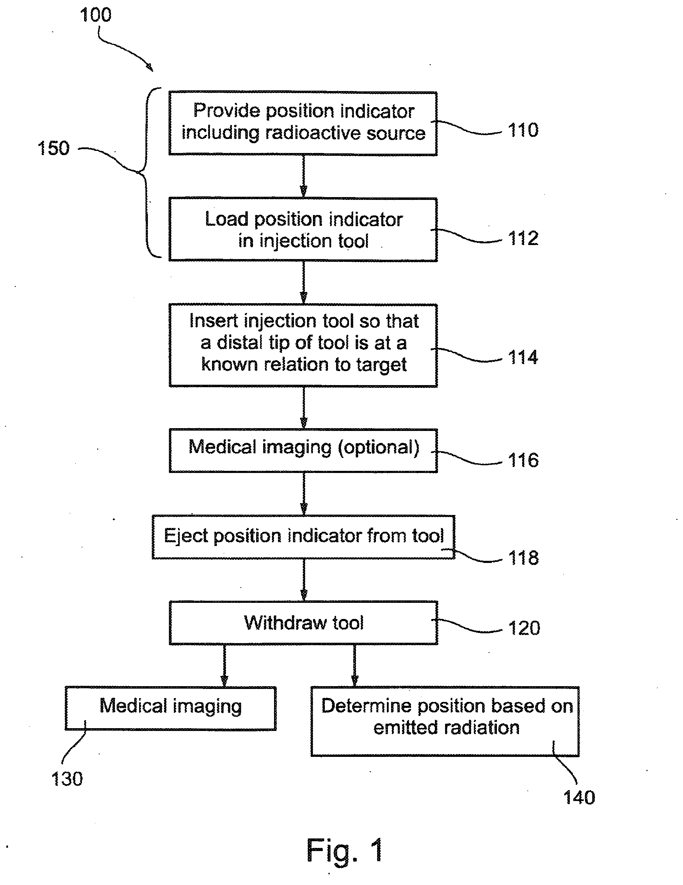

Fig. 1 is a simplified flow diagram of an implantation procedure 300

At 110 an implantable marker including a radioactive source adapted to function as a marker is provided. The marker may comprise a wire. Optionally, the marker is longer than an injection tool into which it will be loaded for injection. The wire may be coiled, folded or disorganized (e.g. jumbled) and then compressed to facilitate loading into the injection tool. Optionally, a degree of coiling folding or jumbling varies along an axial length of the marker. Disorganized sections may be axially distributed along the marker at intervals, optionally regular intervals.

At 112, the marker is loaded into an injection tool. 150 indicates that 110 and 112 may optionally be performed at a manufacturing facility so that the marker is provided as an individually wrapped sterilized unit loaded into an injection tool. Alternatively, the marker and injection tool can be provided as a kit so that loading 112 is performed just prior to insertion 114.

At 114, the injection tool is inserted so that a distal tip of the tool is at a known displacement from the target. Optionally the known displacement is small and the distal tip of the tool approaches a boundary of the target. Optionally the distal tip of the tool is within the target.

116 indicates that insertion 114 may optionally be guided and/or evaluated by medical imaging. Guidance for placement and/or post placement evaluation of relative positions of the marker and the target may be conducted, for example, by ultrasound, fluoroscopy, standard X-ray imaging, CT, MRI or any other available imaging means.

At 118, the marker is ejected from the injection tool. Optionally, ejection is a process whereby the indicator is advanced as the needle is retracted. Optionally, the needle is held in place as a stylet inserted into the needle ejects the marker. Optionally, ejection is at a location which has been evaluated by imaging 116. The marker expands to define a volume whose smallest dimension is larger than an inner diameter of the injection tool (e.g. needle) at this stage.

At 120, the injection tool is withdrawn.

Medical imaging 130 and/or determination of position 140 based upon emitted radiation are performed before and/or after withdrawal 120 of the injection tool. Optionally, imaging 130 and imaging 116 are performed using a same, or a different, imaging modality (e.g. X-ray, CT, MRI or Ultrasound).

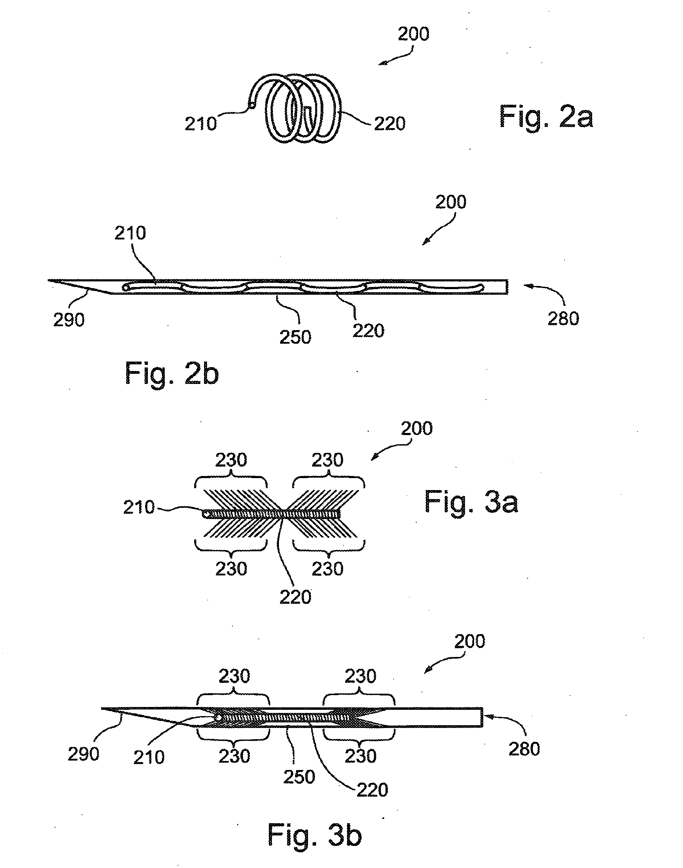

Figs. 2a, 2b 3a and 3d illustrate provision 110 and loading 112 in the context of a coiled (Figs. 2a and 2b) and a herring bone (Figs. 2c and 2d) exemplary configuration of implantable markers 200, not within the scope of claim 1.

Figs. 2A and 3a are schematic representations of implantable markers, not within the scope of claim 1. In the figures , marker 200 comprises a radioactive source 210 and a radio-opaque portion 220. Optionally, radio-opaque portion 220 serves as a fixation element. Optionally, additional anchoring structures 230 (Fig. 3a) are included. Marker 200 may be coated with a biocompatible coating. Optionally, the coating renders marker 200 inert with respect to the body. Preferably implantation of marker 200 does not elicit an immune and/or inflammatory response.

Fig. 2a illustrates a spiral configuration. Optionally, the spiral configuration serves to anchor marker 200 in the body after it is deployed at a desired location. The spiral may be characterized by an elastic memory so that it tends to resume its spiral shape. For example, radio-opaque portion 220 is configured as a spiral and radioactive source 210 is concentrated at one end of marker 200. Radioactive source 210 may also be concentrated in a different location with respect to the spiral or diffused along the spiral.

The radioactive material may be positioned so that the radioactive emissions are substantially spherically uniform.

In Fig. 3a, a straight configuration is illustrated. Optionally, a herringbone pattern of filaments 230 characterized by an elastic memory (or super-elastic, or shape-memory) serves to anchor marker 200 in the body after it is deployed at a desired location. In the figure, radio-opaque portion 220 is configured as a straight cylinder and radioactive source 210 is concentrated at one end of marker 200. Radioactive source 210 may also be concentrated in a different location with respect to the cylinder or diffused along the cylinder. Radioactive source 210 may be a radioactive coating over a non-radioactive material.

Figs. 2B and 3b are schematic representations of the markers depicted in Figs. 2A and 3a respectively loaded in an injection needle 250. Needle 250 may be a standard hypodermic needle, for example a 20 to 25 gauge needle.

Fig. 2B illustrates the compression of spiral portion 220 to a kinked straight configuration within needle 250.

Fig. 3b illustrates the compression of the herringbone pattern of filaments 230 within a needle 250. Application of an ejection force (e.g. from an inserted ejection tool) from proximal side 280 causes ejection of source 200 from distal aperture 290. Elastic memory of relevant portions of source 200 causes the ejected source to tend to revert to the relevant uncompressed configuration. An ejection force may be supplied by an ejection tool and/or by a stream of liquid.

Exemplary Solid Markers

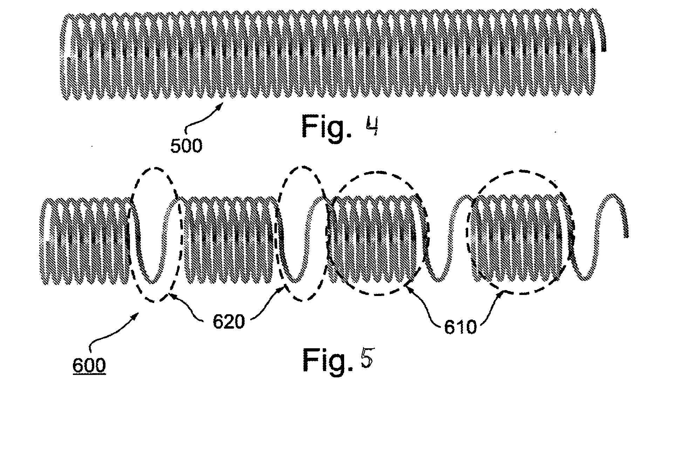

Figs. 4 and 5, illustrate different exemplary configurations of solid markers according to the invention.
The goal of all of the embodiments illustrated in figures 4-5 is to construct a marker that contains sufficient radioactive and radio-opaque material so that it is detectable by a radiation detector and is visible in a medical image. The marker is injectable through a narrow needle ( 20, 21, 23, 27, 27 or intermediate gauges). Optionally, the marker has a pre-injection length of no more than 2-3cm (for ease of injection). According to the invention, the marker is flexible enough to crumple into a 2-5mm diameter spherical or spheroid or ellipsoid shape upon injection into soft tissue.
Fig. 4 depicts an implantable marker 500 configured as a spring. Marker spring 500 can have a wide variety of different dimensions. In general, one or more of spring diameter, pitch and wire diameter can be adjusted in accord with an intended use of the marker. Marker spring 500 may be at least partly constructed of wire containing a gamma emitting radioisotope.
Optionally, a diameter of spring 500 is in the range of 0.2-0.6mm or lesser or greater or intermediate numbers of mm.
Optionally, a pitch of spring 500 is in the range of 0.01mm and 0.2mm or lesser or greater or intermediate numbers of mm.
Optionally, spring 500 is constructed from wire with a diameter in the range of 0.01-0.05mm in diameter or lesser or greater or intermediate numbers of mm.
Fig. 5 depicts an exemplary implantable marker spring 600 which is similar to spring 500 except that it has alternating sections 610 and 620 with different pitches. Optionally, the tight sections 610 have a pitch between 0.01mm and 0.2mm or lesser or greater or intermediate numbers of mm. Optionally, sections 620 are loose and have a pitch between 0.2 and 1mm or lesser or greater or intermediate numbers of mm. Loose sections 620 are spaced between tight sections 610 and contribute to a tendency of marker spring 600 to deform into a spherical, spheroid or ellipsoid shape upon implantation in soft tissue. The lengths of sections 610 and/or 620 are optionally in the range of 0.1mm to 1mm or lesser or greater or intermediate numbers of mm.

Exemplary Radiation Sensors

The implantable marker broadcasts its location radially outward as photons resulting from radioactive disintegrations. Optionally, a portion of this broadcast is received by one or more directional sensors deployed for that purpose. Exemplary sensors and corresponding performance data are described in co-pending PCT applications WO 2006/016368 filed on August 11, 2005 and entitled "Localization of a Radioactive Source within a Body of a Subject" and PCT/IB2006/052770 filed August 10, 2006 and entitled "Localization of a Radioactive Source",

Exemplary Considerations for Visibility in Medical Imaging

In an exemplary embodiment of the invention, the implantable marker is constructed and/or sized to be clearly visible in a medical image of one or more selected types (e.g. x-ray, fluoroscopy, CT, MRI and Ultrasound) Optionally, the marker does not induce significant streaking artifacts in CT images so that use of an implantable marker does not detract from a diagnostic and/or radiation therapy planning value of the image(s).
In order to achieve visibility without introducing undesired artifacts, there is generally a trade off between radio-opacity and the dimensions of the marker. In x-ray imaging (including CT and fluoroscopy), the more radiation the marker absorbs the more clearly visible it is in the image so that use of a material with a higher absorption coefficient allows visibility of a marker with smaller dimensions.
On the other hand, in some imaging modalities (e.g. CT), too much absorption can cause artifacts (e.g. streaks).
In an exemplary embodiment of the invention, a marker that absorbs about two to ten times the amount of radiation as bone and is between 2mm and 5mm in diameter (absorbing between about 2% and about 25% of the radiation, depending on the energy level) provides an acceptable level of visibility in both x-ray and CT images. This physical configuration is typically not large enough to interfere in a clinically significant way with the soft tissue into which it is implanted.
Optionally, the marker comprises primarily a metal body, such as a wire. In those embodiments of the invention in which the marker comprises a continuous metal material deployed in a tissue, a degree of radio-opacity can be adjusted by controlling a volume throughout which the marker is deployed and/or a percentage of the volume filled by the metal material. In an exemplary embodiment of the invention, the implanted marker defines a volume of 2 to 5 mm diameter (or intermediate diameters) within the soft tissue but physically occupies only 5, 10, 15, 25, 50 or 75% or lesser or intermediate percentages of the defined volume.

According to the invention the marker is composed of a coil which folds upon itself during implantation. Optionally, a density of metal within the coil is adjusted by adjusting one or more of wire diameter, coil diameter and pitch of the coil. Optionally, a percentage of the volume defined by the marker which is actually occupied by metal of the marker depends on one or more factors including, but not limited to, mechanical properties of the coil, the implantation tool, and properties of the tissue surrounding the marker. Interaction between these factors determines how the marker folds upon itself when released from the implantation tool. In an exemplary embodiment of the invention, all of these factors are considered when preparing a marker in order to achieve appropriate radio-opacity for x-ray and/or CT visibility.

Once markers have been produced according to theoretical considerations it is possible to test their actual behavior experimentally. Testing can be, for example, by injection into a block of gelatin with a density close to a density of a relevant soft tissue. Gelatin can be sufficiently transparent to allow visual assessment of marker configuration after injection. Optionally, marker configuration is observed for a period of time corresponding to a proposed implantation duration, or longer. Alternatively, or additionally, testing can be performed by injection *in situ* into a piece of a relevant soft tissue removed from a body. Optionally, animal tissue (e.g. rat, rabbit, chicken, pig, or dog) is used as a model for a corresponding human tissue. Alternatively, or additionally, testing can be performed *in vivo* using animals.

Chicken liver may serve as a model for human liver.

As a non-limiting example of theoretical marker design, an exemplary embodiment of the invention, in which a coil made of an alloy of 90% Platinum and 10% Iridium is fashioned into a marker is presented in detail. The exemplary Platinum/Iridium marker can be designed to have a desired level of radio-opacity according to the following procedure.

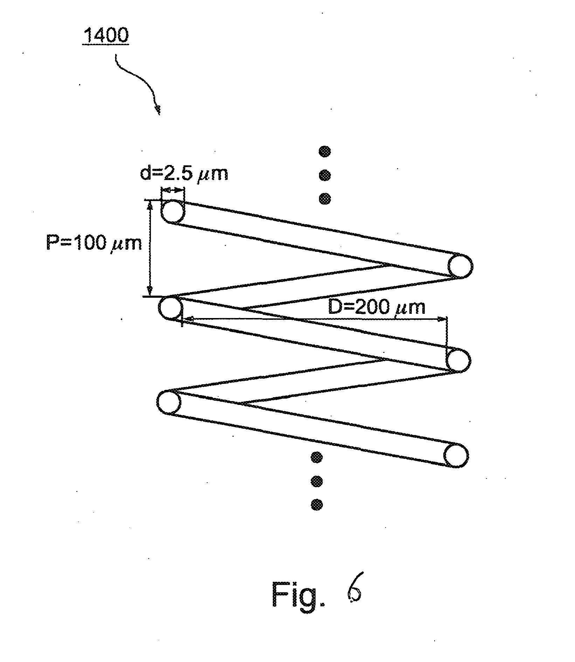

The metal percentage by volume of a coil made from wire with a diameter of 25 microns having an inner coil diameter of 200 microns and a pitch of 100 microns (Fig. 6 shows a coil with these exemplary dimensions) can be calculated as follows:

The total volume of a single turn of the coil is

$$\pi \times \left( \frac{D+2d}{2} \right)^2 \times P = \pi \times 125^2 \times 100 = 4908738 \mu m^3 \qquad \text{EQUATION 1}$$

The length of wire in a single turn of the coil is

$$\frac{\pi \times (D+d)}{\cos\left( \arctan\left( \frac{P}{\pi \times (D+d)} \right) \right)} = \frac{\pi \times 225}{\cos\left( \arctan\left( \frac{100}{\pi \times 225} \right) \right)} = 713.9 \mu m \qquad \text{EQUATION 2}$$

The volume of wire in a single turn of the coil is therefore

$$713.9 \times \pi \times \left( \frac{d}{2} \right)^2 = 713.9 \times \pi \times 12.5^2 = 350435 \mu m^3 \qquad \text{EQUATION 3}$$

The metal percentage by volume is then calculated as the volume of the wire in a single turn of the coil divided by the total volume of a single turn of the coil

$$\frac{350435}{4908738} = 0.07 = 7\% \qquad \text{EQUATION 4}$$

Such a coil has been found in in-situ animal studies to fold upon itself when slowly implanted through a standard 25 gauge biopsy needle into soft tissue, for example liver, so that the coil occupies about 10% of the volume throughout which it disperses. In this case, the metal percentage by volume of the implanted marker is therefore

$$7\% \times 10\% = 0.07 \times 0.1 = 0.007 = 0.7\% \qquad \text{EQUATION 5}$$

The average path length through a sphere is 66.86% of its diameter. The average path length through a 5mm diameter sphere is therefore 3.34mm. The average photon passing through a 5mm diameter sphere whose volume is 0.7% metal will therefore pass through

$$3.34mm \times 0.007 = 0.023mm \text{ of metal.} \qquad \text{EQUATION 6}$$

The mass energy absorption coefficient, $\mu_{en}$, of 10% Iridium / 90% Platinum at 70keV can be calculated as follows:

$$\frac{\mu_{en}}{\rho} = \sum_i W_i \left(\frac{\mu_{en}}{\rho}\right)_i = 0.1 \times 2.07 + 0.9 \times 2.14 = 2.133 \quad \text{EQUATION 7}$$

Where:

$W_i$ is the percentage by weight of the $i^{th}$ component (0.1 for Iridium and 0.9 for Platinum):

$\dfrac{\mu_{en}}{\rho}$ of Iridium at 70keV is 2.07: and

$\dfrac{\mu_{en}}{\rho}$ of Platinum at 70keV is 2.14.

The density of 10% Iridium / 90% Platinum is calculated by

$$\rho = \frac{1}{\sum_i w_i \left(\frac{1}{\rho_i}\right)} = \frac{1}{0.1 \times \frac{1}{22.42} + 0.9 \times \frac{1}{21.45}} = 21.54 \qquad \text{EQUATION 8}$$

Where:

p of Iridium is 22.42g/cm$^3$; and
p of Iridium is 21.45g/cm$^3$.

The $\mu_{en}$, of 10% Iridium / 90% Platinum is then found to be

$$\mu_{en} = \left(\frac{\mu_{en}}{\rho}\right) \times \rho = 2.133 \times 21.54 = 45.94 \qquad \text{EQUATION 9}$$

The average percentage absorption of 70keV photons passing through the marker can then be calculated as:

$$1 - e^{-\mu_{en}x} = 1 - e^{-45.94*0.0023} = 0.1 = 10\% \qquad \text{EQUATION 10}$$

where x is the total thickness of metal that the average photon passed through in centimeters. This is an average, based on the simplification of assuming that the metal in the marker is uniformly distributed throughout the volume occupied by the marker. This is a reasonable approximation given the large size of the pixels/voxels of the images relative to the size of the contortions of the crumpled marker.

This percentage absorption can be increased or decreased as necessary produce a desired visibility in X-ray or CT images by adjusting the size of the marker. Optionally, size adjustment can be achieved by adjusting one or more of a length of the coil that is implanted, a thickness of the wire, a diameter of the coil and the pitch of the coil.

One of ordinary skill in the art will easily be able to modify calculations presented above to account for such adjustments in order to calculate a resulting absorption of the modified marker. Alternatively, or additionally, equations presented above, and others derived therefrom, can be used to perform an inverse optimization calculation in which a desired absorption characteristic is an input and one or more geometric parameters of the marker are calculated.

One of ordinary skill in the art will be able, using the above calculations as a guide, to develop parallel equations for markers with different geometries in order to calculate the percentage of radiation absorbing material within the volume of the marker and then to calculate the percentage of photons that will be absorbed. Alternatively or additionally, a numerical simulation may be used.

Exemplary Radiation Parameters

In an exemplary embodiment of the invention, gamma energies in the range of 100-500kev are high enough energy to escape the body, while being low enough energy to be captured efficiently by radiation detectors and not cause unnecessary radiation damage to the patient.

The level of radioactivity necessary to provide sufficient photons at the detectors depends on the properties of the selected radioisotope, including the number of photons emitted per decay and the energy of the emitted photons. For Iridium-192, for example, an activity in the range of 0.01-0.1mCi provides the appropriate number of photons depending on the distance between the detectors and the patient and on the required tracking performance for the particular application.

In an exemplary embodiment of the invention, Iridium-192 is used in the implantable marker because it is already approved for use in medical applications and is generally considered safe to introduce into the body of a subject. However this isotope is only an illustrative example, and should not be construed as a limitation of the invention. When choosing an isotope for use in an implantable marker according to an exemplary embodiment of the invention, activity (DPM), type and energy of radiation and/or half life may be considered. It is generally desired that disintegration events be detectable with reasonable efficiency at the relevant distance, for example 20-50 cm. Long half lives may be preferred because they make inventory control easier and reduce total costs in the long run by reducing waste. However, short half lives may reduce concerns over radioactive materials and/or may allow smaller sources to be used.

Exemplary Half Life Considerations

In an exemplary embodiment of the invention, the implantable marker includes Iridium (IR [192]). Iridium is characterized by a half life of 73.8 days. According to exemplary embodiments of the invention, isotopes with a half life of 30, optionally 50, optionally 70, optionally 90 days optionally 270 days or greater or intermediate half lives are employed in the implantable marker. In an exemplary embodiment of the invention, these isotopes are compatible with a therapy regimen that lasts 2, optionally 4, optionally 8, optionally 10, optionally 12 weeks or lesser or intermediate or greater numbers of weeks. Exemplary therapies for which implanted trackable radioactive markers may be relevant include, but are not limited to, external beam radiation therapy, proton therapy, biopsy, laparoscopy, minimally invasive surgery, and

robotic surgery.

<u>Safety</u>

The implantable marker may be left in place at the end of the treatment. Optionally, an amount of radiation from the marker is low enough and/or a half life of an isotope included the marker is short enough that there is no significant danger to the patient.

In an exemplary embodiment of the invention, the marker is constructed of biocompatible materials. Optionally, the biocompatible materials are resorbable materials. Optionally, the biocompatible materials comprise an inert coating. Optionally, the inert coating reduces a tendency of the marker to elicit an immune or inflammatory response and/or reduces a tendency of marker components to disperse in the body.

<u>General</u>

A variety of numerical indicators have been utilized to describe the implantable medical marker, radiation from the marker, radiation energy and/or relationships between the implantable marker and surrounding tissue. It should be understood that these numerical indicators could vary even further based upon a variety of engineering principles, materials, intended use and designs incorporated into the invention.

The examples given above are exemplary in nature and are not intended to limit the scope of the invention which is defined solely by the following claims.

The terms "include", "comprise" and "have" and their conjugates as used herein mean "including but not necessarily limited to".

**Claims**

1. An implantable medical marker comprising:

a marker body, adapted for insertion via a needle having a diameter range of 20 to 27 gauge, the body having a tendency to fold on itself multiple times after insertion to define a spheroid-like volume with a smallest dimension larger than an inner dimension of the needle, and a largest dimension between 2 mm and 5 mm; and
a radiation source producing gamma emissions sufficient to exit the human body;
the implantable marker **characterized in that** it comprises a coil.

2. The implantable marker of claim 1, wherein the coil comprises a chain including a plurality of segments of coil having a predetermined pitch, connected by intervening sections of coil having a larger pitch than said predetermined pitch that are adapted to form the folds.

3. The implantable marker of any one of the preceding claims, wherein the gamma emissions produce between $1 \times 10^5$ and $3 \times 10^8$ photons/second.

4. The implantable marker of any one of the preceding claims, wherein the gamma radiation is **characterized by** an average energy of at least 50 kev.

5. The implantable marker of any one of the preceding claims, wherein the implantable marker is **characterized by** at least 1% absorption of an incident X-ray beam on the defined volume.

6. The implantable marker of any one of the preceding claims, wherein the implantable marker induces a radiation dose not exceeding 100 Gy at a distance of 2mm from the implantable marker in 6 months.

7. The implantable marker of any one of the preceding claims, wherein a center of opacity and a center of radioactivity are both within the defined spheroid-like volume.

8. The implantable marker of any one of the preceding claims, **characterized by** an angular distribution of radiation emission from the implantable marker for which the maximum emission in any direction differs by no more than 30% from the minimum emission in any direction.

9. The implantable marker of any one of the preceding claims, **characterized in that** at least a portion of the implantable marker is adapted to absorb between 2 and 25 percent of 70 kev X-ray radiation incident on the implantable marker.

10. The implantable marker of any one of the preceding claims, wherein the marker body is adapted for insertion via a 23 gauge needle.

11. The implantable marker of any one of the preceding claims, wherein the marker body is constructed of a radio-opaque radioactive metal.

12. The implantable marker of any one of the preceding claims, wherein the marker body comprises a radio-opaque radioactive wire with a spring-like memory.

13. The implantable marker of any one of the preceding claims, wherein the implantable marker includes a Platinum/ Iridium alloy.

14. A kit comprising the implantable marker of any one of the preceding claims and an insertion tool.

15. A method of preparing the implantable medical marker of claim 1, the method comprising:

associating an amount of gamma radiation with a metal wire;
forming the wire into a desired shape; and
inserting the wire in the needle.

**Patentansprüche**

1. Implantierbarer medizinischer Marker, umfassend:

einen Markerkörper, ausgelegt für die Einführung über eine Nadel mit einem Durchmesser im Bereich von 20 bis 27 Kaliber, wobei der Körper dazu tendiert, sich nach der Einführung mehrere Male auf sich selbst zu falten, um ein Kugel-ähnliches Volumen zu definieren, wobei die kleinste Abmessung größer als eine Innenabmessung der Nadel ist, und eine größte Abmessung zwischen 2 mm und 5 mm beträgt; und
eine Strahlungsquelle, die Gammastrahlungen erzeugt, die ausreichend sind, um aus dem menschlichen Körper auszutreten;
wobei der implantierbare Marker **dadurch gekennzeichnet ist, dass** er eine Spule umfasst.

2. Implantierbarer Marker nach Anspruch 1, wobei die Spule eine Kette umfasst, die eine Vielzahl von Spulenabschnitten mit einem vorbestimmten Abstand umfasst, verbunden durch vermittelnde Spulenabschnitte mit einem größeren Abstand als dem vorbestimmten Abstand, die ausgelegt sind, um die Faltungen zu bilden.

3. Implantierbarer Marker nach einem der vorhergehenden Ansprüche, wobei die Gammastrahlungen zwischen 1 x $10^5$ und 3 x $10^8$ Photonen pro Sekunde erzeugen

4. Implantierbarer Marker nach einem der vorhergehenden Ansprüche, wobei die Gammastrahlung durch eine durchschnittliche Energie von mindestens 50 KeV gekennzeichnet ist.

5. Implantierbarer Marker nach einem der vorhergehenden Ansprüche, wobei der implantierbare Marker durch mindestens 1 % Absorption eines einfallenden Röntgenstrahls auf dem definierten Volumen ist.

6. Implantierbarer Marker nach einem der vorhergehenden Ansprüche, wobei der implantierbare Marker eine Strahlungsdosis induziert, die 100 Gy bei einem Abstand von 2 mm vom implantierbaren Marker in 6 Monaten nicht übersteigt

7. Implantierbarer Marker nach einem der vorhergehenden Ansprüche, wobei ein Zentrum der Trübung und ein Zentrum der Radioaktivität beide innerhalb des definierten Kugel-ähnlichen Volumens liegen.

8. Implantierbarer Marker nach einem der vorhergehenden Ansprüche, **gekennzeichnet durch** eine winklige Verteilung der Strahlungsemission aus dem implantierbaren Marker, für den die maximale Strahlung in jeder Richtung um nicht mehr als 30 % von der maximalen Strahlung in jeder Richtung abweicht.

**9.** Implantierbarer Marker nach einem der vorhergehenden Ansprüche, **dadurch gekennzeichnet, dass** mindestens ein Abschnitt des implantierbaren Markers ausgelegt ist, um zwischen 2 und 25 Prozent 70 KeV Röntgenstrahlung, die auf den implantierbaren Marker einfällt, zu absorbieren,

**10.** Implantierbarer Marker nach einem der vorhergehenden Ansprüche, wobei der Markerkörper für eine Einführung über eine 23-Kaliber-Nadel ausgelegt ist.

**11.** Implantierbarer Marker nach einem der vorhergehenden Ansprüche, wobei der Markerkörper aus einem strahlenundurchlässigen radioaktiven Metall konstruiert ist.

**12.** Implantierbarer Marker nach einem der vorhergehenden Ansprüche, wobei der Markerkörper einen strahlenundurchlässigen radioaktiven Draht mit einem Federähnlichen Speicher umfasst,

**13.** Implantierbarer Marker nach einem der vorhergehenden Ansprüche, wobei der implantierbare Marker eine Platin/Iridium-Legierung umfasst.

**14.** Kit, umfassend den implantierbaren Marker nach einem der vorhergehenden Ansprüche und ein Einführungswerkzeug.

**15.** Verfahren zur Herstellung des implantierbaren medizinischen Markers nach Anspruch 1, wobei das Verfahren Folgendes umfasst:

Assoziieren einer Menge von Gammastrahlung mit einem Metalldraht;
Bilden des Drahts in einer gewünschten Form; und
Einführen des Drahts in die Nadel.

**Revendications**

**1.** Marqueur médical implantable comprenant :

un corps de marqueur, adapté pour l'insertion via une aiguille ayant un diamètre avec un calibre de l'ordre de 20 à 27, le corps ayant tendance à se replier sur lui-même plusieurs fois après l'insertion pour définir un volume de type sphéroïde avec la plus petite dimension qui est supérieure à une dimension interne de l'aiguille, et la plus grande dimension comprise entre 2 mm et 5 mm ; et
une source de rayonnement produisant des émissions gamma suffisantes pour sortir du corps humain ;
le marqueur implantable étant **caractérisé en ce qu'**il comprend une bobine.

**2.** Marqueur implantable selon la revendication 1, dans lequel la bobine comprend une chaîne comprenant une pluralité de segments de bobine ayant un pas prédéterminé, raccordés par des sections d'intervention de bobine ayant un plus grand pas que ledit pas prédéterminé, qui sont adaptées pour former les plis.

**3.** Marqueur implantable selon l'une quelconque des revendications précédentes, dans lequel les émissions gamma produisent entre $1 \times 10^5$ et $3 \times 10^8$ photons/seconde.

**4.** Marqueur implantable selon l'une quelconque des revendications précédentes, dans lequel le rayonnement gamma est **caractérisé par** une énergie moyenne d'au moins 50 kev.

**5.** Marqueur implantable selon l'une quelconque des revendications précédentes, dans lequel le marqueur implantable est **caractérisé par** au moins 1% d'absorption d'un faisceau de rayons X incident sur le volume défini.

**6.** Marqueur implantable selon l'une quelconque des revendications précédentes, dans lequel le marqueur implantable induit une dose de rayonnement ne dépassant pas 100 Gy à une distance de 2 mm du marqueur implantable en 6 mois.

**7.** Marqueur implantable selon l'une quelconque des revendications précédentes, dans lequel un centre d'opacité et un centre de radioactivité sont tous deux dans le volume de type sphéroïde défini.

**8.** Marqueur implantable selon l'une quelconque des revendications précédentes, **caractérisé par** une distribution angulaire d'émission de rayonnement depuis le marqueur implantable pour laquelle l'émission maximum dans n'importe quelle direction ne diffère pas plus de 30% de l'émission minimum dans n'importe quelle direction.

**9.** Marqueur implantable selon l'une quelconque des revendications précédentes, **caractérisé en ce qu'**au moins une partie du marqueur implantable est adaptée pour absorber entre 2 et 25 pour cent de rayonnement de rayons X à 70 kev incident sur le marqueur implantable.

**10.** Marqueur implantable selon l'une quelconque des revendications précédentes, dans lequel le corps de marqueur est adapté pour l'insertion via une aiguille de calibre 23.

**11.** Marqueur implantable selon l'une quelconque des revendications précédentes, dans lequel le corps de marqueur est construit avec un matériau radioactif radio-opaque.

**12.** Marqueur implantable selon l'une quelconque des revendications précédentes, dans lequel le corps de marqueur comprend un fil radioactif radio-opaque avec une mémoire de type ressort.

**13.** Marqueur implantable selon l'une quelconque des revendications précédentes, dans lequel le marqueur implantable comprend un alliage platine/iridium.

**14.** Kit comprenant le marqueur implantable selon l'une quelconque des revendications précédentes et un outil d'insertion.

**15.** Procédé pour préparer le marqueur médical implantable selon la revendication 1, le procédé comprenant les étapes consistant à :

associer une quantité de rayonnement gamma avec un fil métallique ;
former le fil dans une forme souhaitée ; et
insérer le fil dans l'aiguille.

100

150

Provide position indicator
including radioactive source — 110

Load position indicator
in injection tool — 112

Insert injection tool so that
a distal tip of tool is at a
known relation to target — 114

Medical imaging (optional) — 116

Eject position indicator from tool — 118

Withdraw tool — 120

Medical imaging

130

Determine position based on
emitted radiation

140

Fig. 1

200

210     220     Fig. 2a

200

210

290     280

250     220

Fig. 2b

200

230     230

210

230     230

220

Fig. 3a

200

230     230

290     210     230     230     280

250

220

Fig. 3b

Fig. 4

500

620

610

600

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6436026 B, Sioshani
- US 20040116767 A, Lebovic
- WO 02078785 A
- WO 2004026111 A
- US 6749555 B, Winkler
- US 20030158515 A, Gonzalez
- US 4215694 A, Isakov
- WO 015476 A, ZMED
- US 20050261570 A, Mate
- US 20050027196 A, Fitzgerald
- WO 0057923 A
- US 20050197564 A, Dempsey
- US 6977504 B
- US 6889833 B
- US 6838990 B
- US 6822570 B
- US 6812842 B
- WO 2006016368 A
- US 6603124 B, Maublant
- WO 2006004542 A
- WO 0024332 A
- US 6575991 B
- US 6228055 B
- US 6425903 B
- US 6056700 B
- US 6234177 B
- US 6181960 B
- US 6662041 B
- US 6862470 B
- US 6132359 A
- US 5713828 A
- US 5997463 A
- US 6419621 A
- US 6986880 A
- US 6575888 A
- US 7083566 A
- US 6616591 B